# FASCICULE DE BREVET EUROPEEN

(11) **EP 3 129 070 B1**
(45) Date de publication et mention de la délivrance du brevet: **05.04.2023**
(21) Numéro de dépôt: 14717730.7
(22) Date de dépôt: 10.04.2014
(51) Int. Cl.: A61L 2/26, A61L 2/24, A61L 2/00, A61K 35/00

(54) **KIT DE TRAITEMENT D'INACTIVATION VIRALE D'UN FLUIDE BIOLOGIQUE**
KIT ZUR VIRUSINAKTIVIERUNGSBEHANDLUNG EINER BIOLOGISCHEN FLÜSSIGKEIT
KIT FOR VIRAL INACTIVATION TREATMENT OF A BIOLOGICAL FLUID

(43) Date de publication de la demande: 15.02.2017
(73) Titulaire: Finck, Valérie, 2012 Auvernier (CH)
(72) Inventeur: Finck, Valérie, 2012 Auvernier (CH)
(74) Mandataire: Bovard SA Neuchâtel
(86) Numéro de dépôt international: PCT/EP2014/057327
(87) Numéro de publication internationale: WO 2015/154815

(56) Documents cités:
- EP-A1- 1 685 852
- WO-A1-2009/118331
- US-A1- 2008 090 222

## Description

### Domaine technique

La présente invention concerne un kit de traitement d'inactivation virale d'un fluide biologique, comprenant au moins une poche de traitement apte à contenir le fluide biologique relié à au moins une poche de sortie apte à recevoir le fluide biologique traité par l'intermédiaire de tubes de transport du fluide biologique.

La présente invention concerne également un ensemble de traitement automatisé d'inactivation virale d'un fluide biologique, ledit traitement comprenant une étape de traitement du fluide biologique par un mélange solvant/détergent, une étape d'extraction du mélange solvant/détergent, et éventuellement une étape de filtration au travers d'au moins un filtre à bactéries.

### Etat de la technique

L'inactivation virale d'un fluide biologique, tel que le plasma, grâce à la technique du Solvant Détergent (S/D), est une technique utilisée depuis plus de 25 ans dans le domaine industriel avec comme détergent du Triton X-100^{®} et des quantités très importante de plasma (pool géant).

Le plasma inactivé grâce à la technique du S/D, n'a jamais transmis de virus enveloppés tels HIV, Hépatite B et C.

Toutefois, une fois le plasma inactivé, le S/D doit être éliminé. Différentes méthodes ont été développées pour extraire le S/D, et notamment l'utilisation de la chromatographie sur colonne. Toutefois, cette méthode est réservée au traitement en milieu industriel.

Des solutions ont été cherchées pour traiter le plasma dans de plus petites quantités (mini pool). Une solution est notamment décrite dans la demande WO 2009/118331. Cette demande décrit un procédé d'inactivation virale d'un fluide biologique, comprenant une étape de traitement du fluide biologique par un mélange solvant/détergent, dans lequel l'étape d'extraction du mélange S/D est réalisée au moyen d'huile de soja, la séparation du plasma du mélange S/D se faisant par décantation sous le seul effet de la gravité. Le procédé nécessite l'utilisation de nombreuses poches dans lesquelles le plasma circule pour être traité. L'utilisation de ces nombreuses poches à suspendre par un opérateur sur une potence rend les manipulations nombreuses, et augmente la durée du traitement. Le procédé de traitement est généralement réalisé entre 3h30 à 11h. De plus, le contrôle de la séparation des phases est visuel, l'injection du mélange solvant/détergent se fait manuellement, d'où de nombreux risques de manipulation et d'erreurs de dosage. Une autre solution est également décrite dans la demande EP1685852 A1 qui a pour objet un ensemble de sacs jetables comprenant un sac d'inactivation virale, un compartiment interne, une installation d'entrée et une installation de sortie tous deux connectés au compartiment interne, dans lequel le compartiment interne présent dans le sac présente une section ovoïde, et éventuellement au moins un sac en entonnoir et / ou un sac de chromatographie sur colonne, les différents sacs étant connectables l'un avec l'autre, et à l'utilisation dudit système de sacs jetables pour l'inactivation virale des fluides biologiques avec une excellente récupération des protéines. Bien que la solution proposée par la demande semble efficace, celle-ci n'est pas sans présenter des risques d'erreurs de manipulation et semble difficile à mettre en oeuvre.

En conséquence, un but de la présente demande est de proposer une machine permettant un traitement d'inactivation virale d'un fluide biologique sûr, sans risque d'erreur humaine de manipulation.

Le but de la présente invention est de proposer un kit de traitement d'inactivation virale d'un fluide biologique simple et sûr à manipuler. Divulgation de l'invention

A cet effet, et conformément à la présente invention, il est proposé un kit de traitement selon la revendication 1. La présente invention concerne également un ensemble de traitement automatisé d'inactivation virale d'un fluide biologique selon la revendication 5.

Un tel kit et un tel ensemble permettent d'inactiver les virus d'un fluide biologique de manière entièrement automatisée. Les risques d'erreurs humaines sont supprimés.

### Brève description des dessins

L'invention sera mieux comprise à la lecture de la description qui va suivre, d'un mode de réalisation, donné à titre d'exemple et fait en référence aux dessins dans lesquels:
- la figure 1 est une vue en perspective d'un ensemble selon l'invention comprenant une machine sur laquelle est installé un kit de traitement,
- la figure 2 est une vue avant de la machine selon la figure 1, le kit de traitement ayant été retiré, et
- la figure 3 représente une configuration d'un kit de traitement selon l'invention.

### Mode(s) de réalisation de l'invention

La présente demande concerne une machine 1 de traitement automatisé d'inactivation virale d'un fluide biologique.

D'une manière connue en soi, le traitement d'inactivation virale d'un fluide biologique, tel que le plasma sanguin, comprend :
a) une étape de traitement du fluide biologique par un mélange solvant/détergent,
b) une étape d'extraction du mélange solvant/détergent, réalisée notamment au moyen d'une huile, suivie d'une étape de filtration dans au moins un filtre à charbon afin d'adsorber le mélange solvant/détergent, les résidus d'huile et des pyrogènes au travers desdits filtres à charbon,
c) optionnellement une étape de traitement du fluide biologique issu de l'étape b) par un filtre à bactéries.

Le détergent D est un fluide corrosif, et est de préférence du Triton X-45 ^{®}, utilisé à 0.9%-1 % du volume du fluide biologique à traiter. Le solvant S est de préférence du TnBP (tri-n-butyl phosphate), utilisé à 0.9%-1% du volume du fluide biologique à traiter. Il est bien évident que tout autre détergent ou solvant connu de l'homme du métier pour cette application peut être utilisé. On utilise de préférence une part égale de solvant et de détergent. Selon le volume du fluide biologique à traiter, on utilise de préférence 2% du mélange S/D. L'huile est de préférence de l'huile de soja, ou toute autre huile appropriée connue de l'homme du métier, utilisée à 10% du volume du fluide biologique à traiter.

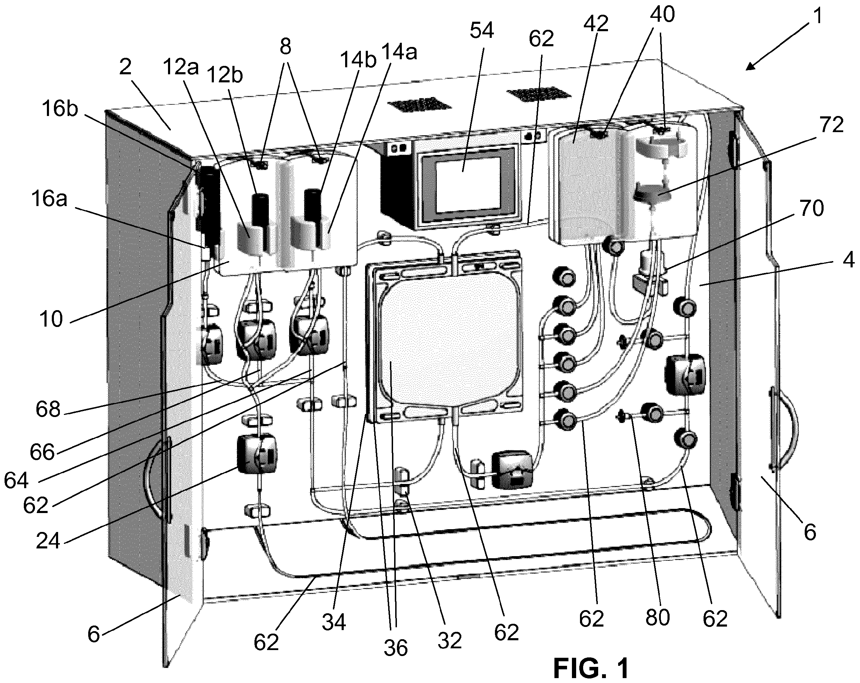

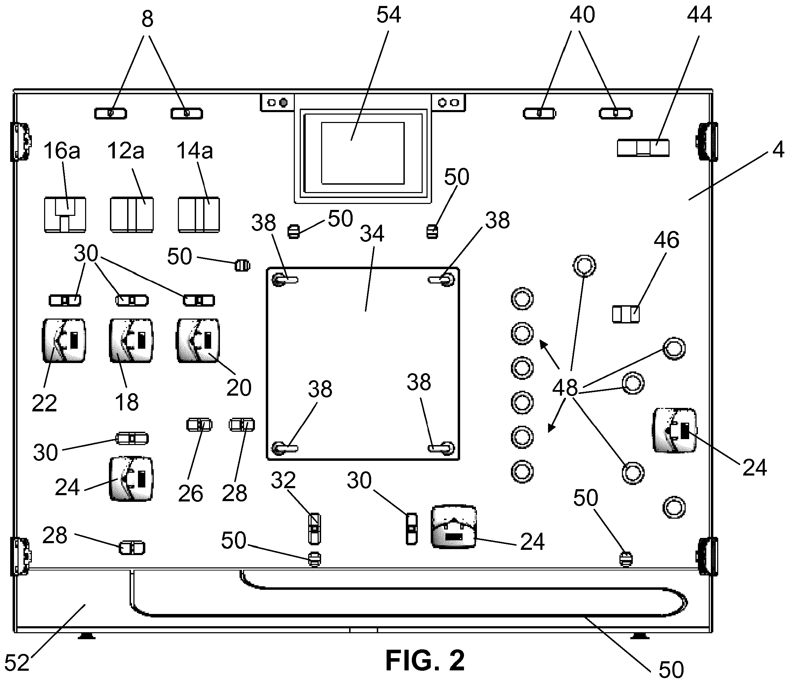

Pour mettre en oeuvre de manière automatisée un tel traitement, et en référence aux figures 1 et 2, la machine 1 de traitement comprend au moins un châssis 2 de forme parallélépipédique, dont la hauteur est supérieure à la profondeur, afin de donner à la machine 1 une configuration verticale lorsqu'elle est posée sur le sol, en position d'utilisation. Le châssis 2 présente un espace intérieur dans lequel sont disposés tous les éléments de la machine tels que décrits ci-dessous. A l'intérieur du châssis 2 est notamment prévue une plaque 4 disposée verticalement et sur laquelle sont montés certains éléments de la machine, comme cela sera décrit ci-après. Les autres éléments et équipements de la machine sont disposés entre le fond du châssis 2 et la plaque 4. Le châssis 2 est fermé par deux portes 6 sécurisées, agencées pour arrêter la machine si les portes sont ouvertes en cours de traitement.

Les éléments montés sur la plaque 4 dans le châssis 2 comprennent notamment deux supports d'entrée 8 destinés à recevoir respectivement au moins une poche d'entrée 10 qui contient le fluide biologique à traiter. Les supports d'entrée 8 sont disposés dans un coin supérieur de la machine, et comprennent notamment un crochet, et la poche d'entrée 10 présente, à son extrémité supérieure, un orifice dans lequel le crochet est introduit pour y suspendre verticalement ladite poche d'entrée 10.

La machine 1 comprend également, fixés sur la plaque 4, un support 12a de flacon de solvant 12b, un support 14a de flacon de détergent 14b, et un support 16a de flacon d'huile 16b, l'huile étant apte à extraire le solvant et le détergent. Les supports de flacon 12a, 14a et 16a sont agencés pour que les flacons correspondants 12b, 14b et 16b soient positionnés verticalement quand la machine est dans sa position d'utilisation, leur ouverture étant orientée vers le bas. Les supports de flacon 12a, 14a et 16a sont positionnés en haut de la plaque 4 de sorte que les poches d'entrée 10 se placent devant les supports de flacon 12a, 14a et 16a.

La machine 1 comprend également, fixés sur la plaque 4, des moyens de circulation du solvant 18, des moyens de circulation du détergent 20, des moyens de circulation de l'huile 22, et des moyens de circulation du fluide biologique 24. De tels moyens de circulation sont par exemple des pompes péristaltiques utilisées pour déplacer les différents liquides d'un point à un autre en circulant dans leurs moyens de transport respectifs, tels que des tubes.

La machine 1 comprend également, fixés sur la plaque 4, des moyens de contrôle du traitement, tels que :
- un capteur de température 26 pour contrôler la température du solvant, du détergent et de l'huile,
- des capteurs de température 28 pour contrôler et réguler la température du fluide biologique, en variant la vitesse des moyens de circulation associés 24. Le fluide biologique passant par la plaque de chauffage 52, il sera de ce fait plus ou moins réchauffé.
- des détecteurs 30, 32 agencés pour contrôler la présence des moyens de transport respectifs des liquides utilisés (solvant, détergent, huile, fluide biologique), la présence, la nature et le volume desdits liquides déplacés. Le détecteur 32 est notamment agencé pour détecter la présence d'huile après décantation.

La machine 1 comprend également, fixé au centre de la plaque 4, un support de traitement 34 destiné à recevoir au moins une, et de préférence deux poches de traitement 36 contenant le fluide biologique. Selon l'invention, ledit support de traitement 34 est positionné sur la plaque 4 du châssis 2 d'une manière à ce que les poches de traitement 36 soient disposées de manière suffisamment verticale pour permettre la séparation du solvant, du détergent et de l'huile ayant aussi capturé du solvant et du détergent, du fluide biologique par décantation sous l'effet de la gravité. Plus précisément, le support de traitement 34 se présente sous la forme d'une plaque positionnée verticalement sur la plaque 4. De plus le support de traitement 34 est monté mobile pour permettre l'agitation verticale du fluide biologique contenu dans les poches de traitement 36. A cet effet, la machine comprend de manière avantageuse, disposé entre le fond du châssis 2 et la plaque 4 un mécanisme de déplacement en translation verticale du support de traitement. Un tel mécanisme est par exemple un chariot (non représenté) sur lequel est fixé le support de traitement 34 de manière verticale, et un système moteur/bielle agencé pour donner au chariot un mouvement de translation verticale bidirectionnel ou alternatif. Ce mouvement permet d'agiter verticalement les poches de traitement 36.

En outre, le support de traitement 34 comprend des crochets 38 permettant la suspension des poches de traitement 36 au moyen d'orifices adéquats prévus aux quatre coins desdites poches de traitement 36. Les poches de traitement 36, reposant sur le support de traitement 34, sont alors positionnées de manière sensiblement verticale. De préférence, le support de traitement 34 est disposé verticalement lorsque la machine est dans sa position d'utilisation, de sorte que les poches de traitement 36 sont verticales, mais il est bien évident que le support de traitement peut être agencé de sorte que les poches de traitement 36 soient légèrement inclinées, l'inclinaison permettant malgré tout une extraction du mélange solvant/détergent du fluide biologique par décantation sous l'effet de la gravité. Ainsi, on peut prévoir que les poches de traitement soient inclinées d'un angle de ± 25° par rapport à la verticale.

La machine 1 comprend également, fixés sur la plaque 4, deux supports de sortie 40 destiné à recevoir respectivement au moins une poche de sortie 42 contenant le fluide biologique traité. Les supports de sortie 40 sont disposés dans le coin supérieur de la machine à l'opposé des supports d'entrée 8, et comprennent notamment un crochet, et la poche de sortie 42 présente, à son extrémité supérieure, un orifice dans lequel le crochet est introduit pour y suspendre verticalement ladite poche de sortie 42.

La machine 1 comprend également, fixés sur la plaque 4, au moins un support de filtre à charbon 44 ainsi qu'au moins un support de filtre à bactéries 46.

La machine 1 comprend également, fixées sur la plaque 4, des vannes à pincement 48 de distribution et/ou de sélection pour autoriser ou non la circulation des différents liquides utilisés, ainsi que des moyens de guidage 50 des moyens de transport desdits liquides. Des moyens de guidage sont par exemple des supports ou des rainures agencés pour recevoir lesdits moyens de transports.

Tous les éléments de la machine fixés sur la plaque 4 sont en conséquence positionnés sur le châssis de manière substantiellement verticale. Cela permet de réaliser toutes les étapes du traitement d'inactivation à la verticale. Ainsi, toutes les poches étant positionnées verticalement durant toute la durée du traitement, il n'est plus nécessaire de prévoir des moyens pour retourner les poches pour l'étape d'extraction du mélange solvant/détergent du fluide biologique par décantation.

La machine1 comprend en outre des moyens de chauffage 52, tels que des plaques de chauffage prévues à la base du châssis, afin d'assurer le maintien d'une température du fluide biologique et de l'environnement (S/D, huile) à 28°C (+/- 3°C) tout au long du process.

La machine 1 comprend en outre une unité de contrôle, munie d'un écran 54, et agencée notamment pour :
- vérifier le volume de solvant et de détergent prélevé dans les flacons et injecté en fonction du volume de fluide biologique à traiter,
- gérer de manière automatique les moyens de contrôle du traitement,
- gérer de manière automatique les moyens de circulation du fluide biologique, les moyens de circulation du solvant, les moyens de circulation du détergent et les moyens de circulation de l'huile,
- gérer les durées des étapes de traitement du fluide biologique
- valider le choix du kit en fonction du volume de fluide biologique à traiter.

Plus spécifiquement, l'unité de contrôle fournit à l'utilisateur un panneau de contrôle ergonomique pour piloter la machine. Elle suit de manière séquentielle les différentes étapes du processus, automatise les traitements et guide l'utilisateur.

La présente invention concerne également un kit de traitement d'inactivation virale d'un fluide biologique, destiné à être utilisé dans une machine de traitement automatisé d'inactivation virale d'un fluide biologique telle que décrite ci-dessus.

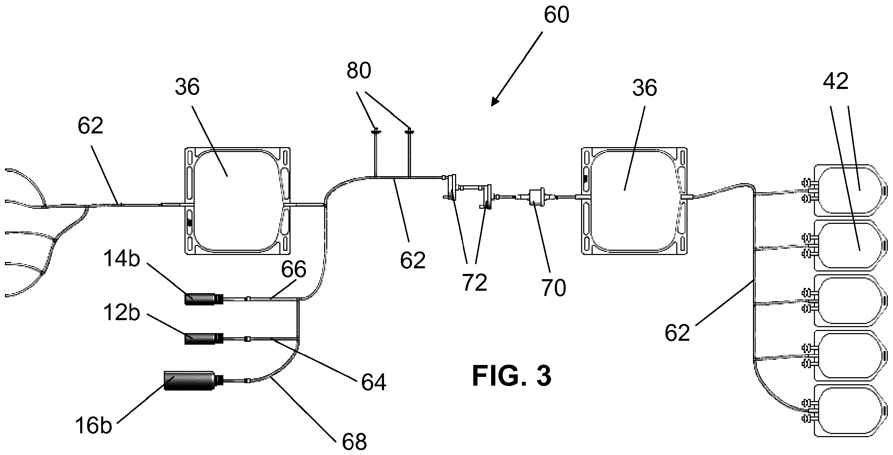

En référence à la figure 3, ledit kit 60 comprend au moins une poche de traitement 36 contenant le fluide biologique, de préférence au moins deux poches de traitement 36, au moins une poche de sortie 42, des moyens de transport 62 du fluide biologique, des moyens de transport 64 de solvant, et des moyens de transport 66 de détergent, des moyens de transport 68 d'huile. Lesdits moyens de transport sont de préférence des tubes permettant de connecter différents éléments entre eux, et dans lesquels circule un liquide. Les poches de traitement peuvent être de toute forme, et ont un volume compris de préférence entre 200ml et 5l. D'une manière avantageuse, le kit 60 comprend également au moins un flacon de solvant 12b, au moins un flacon de détergent 14b, et au moins un flacon d'huile 16b, ledit flacon de solvant 12b, ledit flacon de détergent 14b et ledit flacon d'huile 16b étant à usage unique et contenant respectivement une quantité de solvant, de détergent et d'huile prédosée.

Le kit 60 comprend en outre au moins un filtre à bactéries 70 et/ou au moins un filtre à charbon 72. On utilise de préférence deux filtres à charbon 72. Les filtres à bactérie et à charbon peuvent être tout filtre approprié connu de l'homme du métier et disponible dans le commerce.

D'une manière avantageuse, les flacons 12b, 14b, 16b, de solvant, de détergent et/ou d'huile présentent des bouchons appropriés agencés pour se connecter respectivement aux moyens de transport 64, 66, 68 du solvant, du détergent et de l'huile.

Le kit 60 comprend en outre des arrivées d'air 80 équipées de filtre à air stérile, dont le rôle sera décrit ci-après. Le kit 60 de l'invention est stérile, et à usage unique, y compris les flacons de solvant, détergent et huile. Il est jeté après chaque utilisation. Différentes configurations de kit peuvent être proposées en fonction du volume des poches et du volume du solvant, du détergent et de l'huile. Chaque kit est équipé d'un code d'identification par l'unité de contrôle, tel qu'un code barre.

La machine selon l'invention permet le traitement d'un fluide biologique, tel que le plasma sanguin ou cryoprécipité afin d'en enlever les virus, les bactéries, les pyrogènes, etc... Le plasma peut provenir d'un donneur unique ou de donneurs compatibles, et peut être à 28+/- 3°C. Le plasma traité est ensuite utilisé pour la transfusion.

Lorsque l'utilisateur reçoit les poches d'entrée 10 de fluide à traiter, il les suspend aux supports d'entrée 8 et monte les différents éléments du kit sur la machine en connectant le kit aux poches d'entrée 10 grâce à des connexions stériles. Les flacons de solvant, de détergent et d'huile sont connectés au reste du kit au moyen des bouchons correspondants. L'utilisateur identifie le kit. Le fluide biologique à traiter est pompé dans une première poche de traitement 36 par le haut de ladite poche de traitement 36. L'unité de contrôle vérifie le volume de fluide inscrit par l'opérateur et valide le choix du kit. Le traitement démarre, la température à l'intérieur du châssis étant maintenue à 28 +/- 3°C.

Les pompes correspondant à la circulation du solvant et du détergent sont actionnées, de sorte que ledit solvant et ledit détergent contenus dans leurs flacons séparés sont chargés dans la première poche de traitement 36 par la base de cette dernière. Les différents moyens de contrôle permettent de contrôler le volume de solvant et de détergent injecté. Le support de traitement 34 est actionné pour permettre une agitation verticale de la première poche de traitement. Après l'inactivation des virus, la pompe correspondant à la circulation de l'huile est actionnée, de sorte que l'huile contenue dans son flacon séparé est chargée dans la première poche de traitement 36 par la base de cette dernière. Les moyens de contrôle permettent de contrôler le volume d'huile injecté. L'agitation verticale de la première poche 36 est maintenue par déplacement vertical du support de traitement 34. L'agitation est ensuite arrêtée pour permettre la décantation du milieu, et l'extraction du mélange solvant/détergent du fluide biologique au moyen de l'huile.

Le fluide biologique séparé du solvant et du détergent est pompé à l'aide d'une pompe péristaltique par la base de la première poche de traitement 36 et est amené à la deuxième poche de traitement 36 par le haut de cette dernière, en passant par les filtres à charbon 72 et le filtre à bactérie 70. Dès que le détecteur 32 détecte la présence d'huile, la pompe est arrêtée. De l'air est injecté dans le circuit par les arrivées d'air 80 afin de vider les tubes. Le support de traitement 34 est mis en mouvement afin d'agiter la deuxième poche de traitement 36 de manière verticale le temps nécessaire. Puis le fluide traité est pompé par la base de la deuxième poche de traitement 36 et est réparti dans les différentes poches de sortie 42 au moyen des vannes 48. L'unité de contrôle définit les volumes de remplissage des différentes poches de sortie 42. De l'air est de nouveau injecté dans le circuit pour le vider. Les poches de sortie 42 sont dépendues et scellées. Le kit est démonté et jeté.

La distribution par poches dépendra du volume de fluide biologique initial. Les poches auront un volume minimum de 200 ml et d'autres volumes définis par l'opérateur.

La machine assure un parfait mélange du solvant/détergent et du fluide biologique et une parfaite séparation du fluide biologique de tous les autres constituants (solvant+détergent+huile).

En cas de problème (pas assez de réactifs, problème de température, tuyaux mal mis), la machine comprend des moyens de sécurité permettant d'arrêter le traitement.

On récupère les poches de fluide biologique traité, et on les utilise selon les habitudes du centre de transfusion (congélation ou transfusion).

Le fluide biologique ainsi traité sera transfusé principalement aux patients hémophiles, aux transplantés cardiaques, aux graves traumas, et aux malades souffrant d'une maladie du foie....

D'une manière avantageuse, la durée totale du traitement est de 1h à 2h30.

La machine permet d'obtenir un fluide biologique inactivé, bactériologiquement filtré, sans trace de pyrogènes de solvant ni de détergent. Elle permet également de réduire la durée de traitement pour inactiver le fluide biologique. La machine permet également d'automatiser entièrement le procédé de traitement, depuis l'arrivée des poches de fluide biologique à traiter jusqu'au départ des poches de fluide biologique traité. L'opérateur n'ayant qu'à mettre en place le kit dans la machine et qu'à le retirer à la fin du traitement, toute source d'erreur humaine est supprimée et les temps de manipulation sont fortement réduits.

## Revendications

1. Kit de traitement d'inactivation virale d'un fluide biologique, comprenant au moins une poche de traitement (36) apte à contenir le fluide biologique et reliée à au moins une poche de sortie (42) apte à recevoir le fluide biologique traité par l'intermédiaire de tubes de transport (62) du fluide biologique, **caractérisé en ce qu'**il comprend au moins un flacon de solvant (12b), au moins un flacon de détergent (14b) et au moins un flacon d'huile (16b), lesdits flacons de solvant (12b), de détergent (14b) et d'huile (16b) étant à usage unique et contenant respectivement une quantité de solvant, de détergent et d'huile prédosée, le solvant et le détergent étant destinés à réaliser un mélange de traitement de fluide biologique et l'huile étant destinée à extraire ledit mélange de traitement du fluide biologique traité, lesdits flacons étant reliés respectivement par des tubes de transport (64, 66, 68) de solvant, de détergent, et d'huile à ladite poche de traitement de fluide biologique (36).

2. Kit selon la revendication 1, **caractérisé en ce qu'**il comprend en outre au moins un filtre à charbon (72), et/ou au moins un filtre à bactéries (70) entre la poche de traitement (36) et la poche de sortie (42).

3. Kit selon la revendication 2, **caractérisé en ce qu'**il comporte deux poches de traitement (36), ledit au moins un tefiltre à charbon (72) et/ou ledit au moins un filtre à bactéries (70) étant disposés entre les deux poches de traitement (36) reliées entre elles et au(x) dit(s) filtre(s) à charbon et/ou bactérie par des dits tubes de transport (62).

4. Kit selon l'une des revendications 1 à 3, **caractérisé en ce que** les flacons de solvant, de détergent et/ou d'huile présentent des bouchons agencés pour se connecter respectivement aux moyens de transport du solvant, du détergent et de l'huile.

5. Ensemble de traitement automatisé d'inactivation virale d'un fluide biologique, ledit traitement comprenant une étape de traitement du fluide biologique par un mélange solvant/détergent, une étape d'extraction du mélange solvant/détergent, et optionnellement une étape de traitement par un filtre à bactéries, **caractérisé en ce qu'**il comprend :
- un kit de traitement d'inactivation de fluide biologique selon l'une des revendications précédentes, et
- une machine (1) de traitement automatisé d'inactivation virale d'un fluide biologique, comprenant au moins un châssis (2) présentant un espace intérieur, ledit châssis (2) comprenant au moins un fond et une plaque (4) disposée dans ledit espace intérieur et sur laquelle sont agencés au moins :
- un support d'entrée (8), destiné à recevoir au moins une poche d'entrée (10) contenant le fluide biologique à traiter,
- un support (12a) de flacon de solvant 12(b), un support (14a) de flacon de détergent, et un support (16a) de flacon d'huile (16b), lesdits supports étant agencés sur la plaque (4) de telle sorte que les flacons correspondants du kit de traitement soient positionnés verticalement, ouverture vers le bas, lorsque la machine est en position d'utilisation,
- des moyens de circulation du solvant (18), du détergent (20), et de l'huile (22), et du fluide biologique (24) aptes à déplacer les dits solvant, détergent, huile et fluide biologique dans les tubes de transports respectifs (64, 66, 68) vers la poche de traitement (36) du kit de traitement,
- des moyens de contrôle du traitement,
- un support de traitement (34) destiné à recevoir ladite au moins une poche de traitement (36) du kit de traitement contenant le fluide biologique, ledit support de traitement (34) étant positionné sur la plaque (4) du châssis (2) d'une manière à ce que les poches de traitement (36) ayant un volume compris de préférence entre 200ml et 5l soient disposées de manière suffisamment verticale pour permettre la séparation du solvant et du détergent du fluide biologique par décantation sous l'effet de la gravité après mélange dudit fluide biologique à traiter avec lesdits solvant et détergent, et
- au moins un support de sortie (40) destiné à recevoir au moins la poche de sortie (42) du kit de traitement contenant le fluide biologique traité.

6. Ensemble selon la revendication précédente, **caractérisé en ce que** les moyens de contrôle du traitement de la machine de traitement comprennent des capteurs de température (28).

7. Ensemble selon l'une des revendications 5 ou 6, **caractérisée en ce que** la machine comprend en outre des moyens de chauffage (52).

8. Ensemble selon l'une des revendications 5 à 7, **caractérisé en ce que** la machine comprend, disposé entre le fond du châssis (2) et la plaque (4), un mécanisme de déplacement en translation verticale du support de traitement (34).

9. Ensemble selon l'une quelconque des revendications 5 à 8, **caractérisé en ce que** la machine comprend en outre une unité de contrôle agencée pour vérifier le volume de solvant et de détergent injecté en fonction du volume de fluide biologique à traiter.

10. Ensemble selon la revendication 9, **caractérisé en ce que** l'unité de contrôle de la machine est en outre agencée pour gérer les durées des étapes de traitement du fluide biologique.

11. Ensemble selon l'une des revendications 9 ou 10, **caractérisé en ce que** l'unité de contrôle est en outre agencée pour valider le choix du kit en fonction du volume de fluide biologique à traiter.

## Patentansprüche

1. Kit zur Virusinaktivierungsbehandlung einer biologischen Flüssigkeit, umfassend mindestens ein Behandlungsfach (36), das zum Enthalten der biologischen Flüssigkeit geeignet ist und in Verbindung steht mit mindestens einem Austrittsfach (42), das zum Empfangen der behandelten biologischen Flüssigkeit mittels Transportkanälen (62) der biologischen Flüssigkeit geeignet ist, **dadurch gekennzeichnet, dass** es mindestens eine Lösungsmittelflasche (12b), mindestens eine Detergensflasche (14b) und mindestens eine Ölflasche (16b) umfasst, wobei die Lösungsmittelflasche (12b), die Detergensflasche (14b) und die Ölflasche (16b) zum einmaligen Gebrauch vorgesehen sind und jeweils eine vordosierte Menge des Lösungsmittels, des Detergens und des Öls enthalten, wobei das Lösungsmittel und das Detergens zum Bilden einer Behandlungsmischung der biologischen Flüssigkeit vorgesehen sind und das Öl zum Extrahieren der Behandlungsmischung der behandelten biologischen Flüssigkeit vorgesehen ist, wobei die Flaschen jeweils mit den Transportkanälen (64, 66, 68) des Lösungsmittels, des Detergens und des Öls zu dem Behandlungsfach der biologischen Flüssigkeit (36) verbunden sind.

2. Kit nach Anspruch 1, **dadurch gekennzeichnet, dass** es außerdem mindestens einen Kohlefilter (72) und/oder mindestens einen Bakterienfilter (70) zwischen dem Behandlungsfach (36) und dem Austrittsfach (42) umfasst.

3. Kit nach Anspruch 2, **dadurch gekennzeichnet, dass** es zwei Behandlungsfächer (36) beinhaltet, wobei der mindestens eine Kohlefilter (72) und/oder der mindestens eine Bakterienfilter (70) zwischen den beiden Behandlungsfächern (36) angeordnet sind und zwischen ihnen und dem/den Kohlefiltern und/oder dem/den Bakterienfiltern mittels der Transportkanäle (62) eine Verbindung besteht.

4. Kit nach einem der Ansprüche 1 bis 3, **dadurch gekennzeichnet, dass** die Lösungsmittelflasche, die Detergensflasche und/oder die Ölflasche über geeignete Stöpsel verfügen, um jeweils mit Transportmitteln des Lösungsmittels, des Detergens und des Öls verbunden zu werden.

5. Anordnung zur automatisierten Virusinaktivierungsbehandlung einer biologischen Flüssigkeit, wobei die Behandlung einen Schritt des Behandelns der biologischen Flüssigkeit mit einer Mischung aus Lösungsmittel und Detergens, einen Schritt des Extrahierens der Mischung aus Lösungsmittel und Detergens und gegebenenfalls einen Schritt des Behandelns mit einem Bakterienfilter umfasst, **dadurch gekennzeichnet, dass** sie umfasst:
- ein Kit zur Virusinaktivierungsbehandlung von biologischer Flüssigkeit nach einem der vorhergehenden Ansprüche, und
- eine Maschine (1) zur automatisierten Virusinaktivierungsbehandlung einer biologischen Flüssigkeit, umfassend mindestens ein Gehäuse (2), das einen Innenraum aufweist, wobei das Gehäuse (2) mindestens einen Boden und eine Platte (4) umfasst, die in dem Innenraum angeordnet ist, und auf der mindestens folgendes angeordnet ist:
- ein Einlassträger (8), der zum Empfangen von mindestens einem Einlassfach (10) vorgesehen ist, welches die zu behandelnde biologische Flüssigkeit enthält,
- ein Träger (12a) der Lösungsmittelflasche 12(b), ein Träger (14a) der Detergensflasche und ein Träger (16a) der Ölflasche (16b), wobei die Träger auf der Platte (4) derart eingerichtet sind, dass die Flaschen entsprechend dem Kit zur Behandlung vertikal mit der Öffnung nach unten positioniert sind, wenn sich die Maschine in der Gebrauchsposition befindet,
- Umwälzmittel für das Lösungsmittel (18), das Detergens (20) und das Öl (22) sowie die biologische Flüssigkeit (24), die zum Bewegen des Lösungsmittels, des Detergens, des Öls und der biologischen Flüssigkeit in den jeweiligen Transportkanälen (64, 66, 68) in Richtung auf das Behandlungsfach (36) des Kits zur Behandlung geeignet sind,
- Mittel zur Steuerung der Behandlung,
- ein Behandlungsträger (34), der zum Empfangen des mindestens einen Behandlungsfachs (36) des Kits zur Behandlung vorgesehen ist, welches die biologische Flüssigkeit enthält, wobei der Behandlungsträger (34) auf der Platte (4) des Gehäuses (2) in einer Weise positioniert ist, dass die Behandlungsfächer (36), die ein Volumen haben, das vorzugsweise zwischen 200 ml und 5 1 liegt, in einer Weise angeordnet sind, die ausreichend vertikal ist, um die Trennung des Lösungsmittels und des Detergens von der biologischen Flüssigkeit mittels Dekantierung unter Einwirkung der Schwerkraft zuzulassen, nachdem die biologische Flüssigkeit zur Behandlung mit dem Lösungsmittel und dem Detergens gemischt wurde, und
- mindestens ein Austrittsträger (40), der zum Empfangen von mindestens dem Austrittsfach (42) des Kits zur Behandlung vorgesehen ist, welches die behandelte biologische Flüssigkeit enthält.

6. Anordnung nach dem vorhergehenden Anspruch, **dadurch gekennzeichnet, dass** die Mittel zur Steuerung der Behandlung der Maschine zur Behandlung Temperaturfühler (28) umfassen.

7. Anordnung nach einem der Ansprüche 5 oder 6, **dadurch gekennzeichnet, dass** die Maschine außerdem Heizmittel (52) umfasst.

8. Anordnung nach einem der Ansprüche 5 bis 7, **dadurch gekennzeichnet, dass** die Maschine, angeordnet zwischen dem Boden des Gehäuses (2) und der Platte (4), einen Mechanismus zur translatorischen Vertikalbewegung des Behandlungsträgers (34) umfasst.

9. Anordnung nach einem der Ansprüche 5 bis 8, **dadurch gekennzeichnet, dass** die Maschine außerdem eine Steuereinheit umfasst, welche zum Verifizieren des Volumens des Lösungsmittels und des Detergens geeignet ist, welches in Abhängigkeit von dem Volumen der zu behandelnden biologischen Flüssigkeit injiziert wird.

10. Anordnung nach Anspruch 9, **dadurch gekennzeichnet, dass** die Steuereinheit der Maschine außerdem zum Management der Dauer der Schritte zur Behandlung der biologischen Flüssigkeit geeignet ist.

11. Anordnung nach einem der Ansprüche 9 oder 10, **dadurch gekennzeichnet, dass** die Steuereinheit außerdem zum Validieren der Auswahl des Kits in Abhängigkeit von dem Volumen der zu behandelnden biologischen Flüssigkeit geeignet ist.

## Claims

1. A kit for viral inactivation treatment of a biological fluid, comprising at least one treatment bag (36) suitable for containing the biological fluid and connected to at least one output bag (42) suitable for receiving the treated biological fluid by way of tubes (62) for transporting the biological fluid, **characterised in that** said kit comprises at least one bottle of solvent (12b), at least one bottle of detergent (14b) and at least one bottle of oil (16b), said bottles of solvent (12b), detergent (14b) and oil (16b) being single-use and containing respectively a quantity of solvent, detergent and pre-dosed oil, the solvent and the detergent being intended to produce a mixture for treating biological fluid and the oil being intended to extract said mixture for treating the treated biological fluid, said bottles being connected respectively by tubes (64, 66, 68) for transporting the solvent, detergent and oil to said biological fluid treatment bag (36).

2. The kit according to Claim 1, **characterised in that** it additionally comprises at least one carbon filter (72), and/or at least one bacterial filter (70) between the treatment bag (36) and the output bag (42).

3. The kit according to Claim 2, **characterised in that** it includes two treatment bags (36), said at least one carbon filter (72) and/or said at least one bacterial filter (70) being disposed between the two treatment bags (36) which are connected to one another and to said carbon and/or bacterial filter(s) by said transport tubes (62).

4. The kit according to any one of Claims 1 to 3, **characterised in that** the bottles of solvent, detergent and/or oil have stoppers arranged to be connected respectively to the means for transporting the solvent, the detergent and the oil.

5. An assembly for automated viral inactivation treatment of a biological fluid,
said treatment comprising a step of treating the biological fluid by a solvent/detergent mixture, a step of extracting the solvent/detergent mixture, and optionally a step involving treatment by a bacterial filter, **characterised in that** it comprises:
- a kit for inactivation treatment of a biological fluid according to any one of the preceding claims, and
- a machine (1) for automated viral inactivation treatment of a biological fluid, comprising at least one chassis (2) having an interior space, said chassis (2) comprising at least one bottom and a plate (4) disposed in said interior space and on which are located at least:
- an input mounting (8) intended for receiving at least one input bag (10) containing the biological fluid to be treated,
- a mounting (12a) of a bottle of solvent 12 (b), a mounting (14a) of a bottle of detergent, and a mounting (16a) of a bottle of oil (16b), said mountings being arranged on the plate (4) so that the corresponding bottles of the treatment kit are positioned vertically, opening downwards, when the machine is in the usage position,
- means for circulating the solvent (18), the detergent (20), and the oil (22), and the biological fluid (24) suitable for displacing said solvent, detergent, oil and biological fluid in the respective transport tubes (64, 66, 68) towards the treatment bag (36) of the treatment kit,
- means for monitoring the treatment,
- a treatment mounting (34) intended for receiving said at least one treatment bag (36) of the treatment kit containing the biological fluid, said treatment mounting (34) being positioned on the plate (4) of the chassis (2) in such a manner that the treatment bags (36) having a volume preferably between 200ml and 51 are disposed in a sufficiently vertical manner to allow the separation of the solvent and the detergent from the biological fluid by decanting under the effect of gravity, after mixing said biological fluid to be treated with said solvent and detergent, and
- at least one output mounting (40) intended for receiving at least the output bag (42) of the treatment kit containing the treated biological fluid.

6. The assembly according to the preceding claim, **characterised in that** the means for monitoring the treatment of the treatment machine comprise temperature sensors (28).

7. The assembly according to any one of Claims 5 or 6, **characterised in that** the machine additionally comprises heating means (52).

8. The assembly according to any one of Claims 5 to 7, **characterised in that** the machine comprises, disposed between the bottom of the chassis (2) and the plate (4), a mechanism for displacing the treatment mounting (34) in a vertical movement.

9. The assembly according to any one of Claims 5 to 8, **characterised in that** the machine additionally comprises a monitoring unit arranged to check the volume of solvent and of detergent injected depending on the volume of the biological fluid to be treated.

10. The assembly according to Claim 9, **characterised in that** the monitoring unit of the machine is additionally arranged to manage the durations of the steps of treating the biological fluid.

11. The assembly according to any one of Claims 9 or 10, **characterised in that** the monitoring unit is additionally arranged to validate the choice of the kit depending on the volume of the biological fluid to be treated.
